(19) Europäisches Patentamt / European Patent Office / Office européen des brevets

(11) **EP 4 328 366 A1**

(12) **EUROPÄISCHE PATENTANMELDUNG**

(43) Veröffentlichungstag:
**28.02.2024   Patentblatt 2024/09**

(21) Anmeldenummer: **23190726.2**

(22) Anmeldetag: **10.08.2023**

(51) Internationale Patentklassifikation (IPC):
**D04B 1/10** *(2006.01)*       **D04B 1/18** *(2006.01)*
**D04B 1/22** *(2006.01)*       **A61F 2/78** *(2006.01)*

(52) Gemeinsame Patentklassifikation (CPC):
**D04B 1/22; A61F 2/78; D04B 1/108; D04B 1/18;**
D10B 2403/033; D10B 2509/028

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **22.08.2022   DE 102022121165**

(71) Anmelder: **Julius Zorn GmbH**
**86551 Aichach (DE)**

(72) Erfinder: **Lechner, Siegfried**
**86529 Schrobenhausen (DE)**

(74) Vertreter: **Charrier Rapp & Liebau**
**Patentanwälte PartG mbB**
**Fuggerstraße 20**
**86150 Augsburg (DE)**

(54) **KOMPRESSIONSGESTRICK UND VERFAHREN ZUR HERSTELLUNG EINES KOMPRESSIONSGESTRICKS, INSBESONDERE ZUR HERSTELLUNG VON GESTRICKTEN STUMPFSTRÜMPFEN MIT KOMPRESSIVER WIRKUNG**

(57)     Die Erfindung betrifft ein Verfahren zur Herstellung eines zumindest im Wesentlichen schlauchförmigen Kompressionsgestricks, welches sich von einem offenen proximalen Ende (p) zu einem geschlossenen distalen Ende (d) des Kompressionsgestricks erstreckt, mit folgenden Schritten:
• Stricken eines schlauchförmigen ersten Abschnitts (A1) eines Maschen (m) bildenden Grundgestricks (M) aus wenigstens einem Strickfaden (T) entlang einer Strickrichtung (v), die von dem proximalen Ende (p) zu dem distalen Ende (d) des Kompressionsgestricks verläuft, mit einer Mehrzahl von in Strickrichtung (v) aufeinanderfolgenden Maschenreihen (R), wobei in die Maschen (m) des Grundgestricks (M) in jeder Maschenreihe (R) oder in jeder n-ten Maschenreihe (M) mit $n \in \mathbb{N}$ und $n \geq 2$ ein elastischer Schussfaden (S) über Fanghenkel (F) eingebunden wird,
• Nahtloses Anstricken eines zweiten Abschnitts (A2) an den ersten Abschnitt (A1) des Grundgestricks (M) aus dem wenigstens einen Strickfaden (T) entlang der Strickrichtung (v), wobei in dem zweiten Abschnitt (A2) in Strickrichtung (v) gesehen sukzessive die Anzahl der Maschen (m) zumindest in einigen oder allen aufeinanderfolgenden Maschenreihen (R) durch Umhängen von Maschen (m) vermindert wird, wodurch in dem zweiten Abschnitt (A2) des Grundgestricks (M) zum distalen Ende (d) hin durch Maschenminderung ein kuppelförmig geschlossener Abschluss (A3) ausgebildet wird.

**Fig. 4**

**Beschreibung**

Die Erfindung betrifft ein Verfahren zur Herstellung eines Kompressionsgestricks, insbesondere zur Herstellung von gestrickten Stumpfstrümpfen mit kompressiver Wirkung, sowie ein Kompressionsgestrick, insbesondere zur Herstellung von gestrickten Stumpfstrümpfen mit kompressiver Wirkung.

Aus dem Stand der Technik sind Stumpfstrümpfe zum Anlegen an amputierte Gliedmaßen (Gliedmaßenstümpfe) bekannt, wobei die Stumpfstrümpfe aus einem Kompressionsgestrick gebildet sind, welches eine kompressive Wirkung auf den Gliedmaßenstumpf ausübt, um den Stumpf therapeutisch zu komprimieren. Dadurch kann eine Schwellung des Stumpfes durch Ödeme unterdrückt werden. Um die Blutzirkulation in dem Stumpf nicht zu behindern, muss der vom Stumpfstrumpf ausgeübte Kompressionsdruck genau eingestellt werden. Zur Förderung des lymphatischen Systems in dem Stumpf wird bevorzugt ein Druckverlauf mit einem vom distalen Ende des Stumpfs graduelle abnehmenden Kompressionsdruck eingestellt. Am offenen proximalen Ende des Stumpfstrumpfs erzeugt das Kompressionsgestrick damit einen geringeren Kompressionsdruck als am geschlossenen distalen Ende.

Bekannte Kompressionsgestricke für Stumpfstrümpfe werden bspw. als Rundgestricke auf Rundstrickmaschinen hergestellt. Problematisch ist dabei die Herstellung des kuppelförmigen Abschlusses am distalen Ende des Stumpfstrumpfs. Hierfür wird bei den rundgestrickten Kompressionsgestricken am distalen Ende eine runde Öffnung belassen, die bspw. durch ein kreisförmiges Polster oder eine gestrickte Endkappe geschlossen wird, indem das Polster oder die gestrickte Endkappe angenäht wird. Aufgrund der hierfür benötigten Naht entsteht am distalen Ende des Stumpfstrumpfs eine Verdickung, die einerseits den graduellen Verlauf des Kompressionsdrucks verfälscht und andererseits zu Reibung zwischen dem Stumpfstrumpf und dem Stumpf und dadurch bedingt zu Hautirritation am Stumpf führen kann.

Aus dem Stand der Technik sind ferner Kompressionsgestricke für Stumpfstrümpfe bekannt, die aus zwei, jeweils auf einer Flachstrickmaschine gestrickten Flachgestrickteilen bestehen, die an ihren Seitenrändern entlang einer Längsnaht zusammengenäht werden. Auch hier kommt es durch die Längsnähte zu einer Beeinträchtigung des gewünschten Druckverlaufs und zu Hautirritation am Stumpf und die Längsnähte werden vom Patienten als störend empfunden.

Zur Vermeidung dieser Nachteile bei rundgestrickten oder aus zwei flachgestrickten Gestrickteilen zusammengesetzten Stumpfstrümpfen wurden im Stand der Technik Kompressionsgestricke vorgeschlagen, die nahtlos auf einer Flachstrickmaschine mit zwei gegenüberliegenden Nadelbetten hergestellt werden können. Ein solches Kompressionsgestrick ist aus der US 7 363 778 B2 bekannt, die eine dehnbare Manschette offenbart, die auf einem amputierten Glied mit einem distalen Ende aufgenommen werden kann und sich dem amputierten Glied anpasst, Die dehnbare Manschette umfasst im Wesentlichen eine einheitliche Aufnahme für das amputierte Glied, wobei die Aufnahme im Wesentlichen einen schlauchförmigen Abschnitt und einen abgerundeten Endabschnitt umfasst. Insbesondere umfasst der abgerundete Endabschnitt gegenüberliegende Bahnen, die in Reihe mit einer Vielzahl von verstrickten kurzen Reihen gestrickt sind, die eine progressive Verjüngung von einem offenen Rand des schlauchförmigen Abschnitts zum proximalen Zehende des Endabschnitts schaffen. Die progressive Verjüngung der gestrickten Bahnen ermöglicht es, dass der äußerste Rand des abgerundeten Endabschnitts eine abgerundete Form annimmt, die sich bequem an eine amputierte Gliedmaße anpasst und keine Nähte aufweist, die andernfalls das empfindliche Ende der amputierten Gliedmaße reizen würden.

Die bekannten Kompressionsgestricke für amputierte Gliedmaßen (Gliedmassenstümpfe) sind aus einem elastischen Strickfaden, bspw. einem umwunden Faden mit einem elastischen Elasthan-Kernfaden gestrickt, um dadurch ein dehnbares Gestrick zu erzeugen, welches sich beim Anlegen an das amputierte Glied ausdehnen und dadurch einen von der Dehnung abhängigen Kompressionsdruck ausüben kann. Eine genaue Einstellung eines gewünschten Kompressionsdrucks und insbesondere die Erzeugung eines graduellen Druckverlaufs mit einem vom distalen Ende des Strumpfstrumpfs zum proximalen Ende abnehmenden Kompressionsdruck ist bei diesen Gestricken allerdings schwierig, da der erzeugte Kompressionsdruck von der Dehnung des Gestricks im Umfangsrichtung des Glieds und diese wiederum zum einen von der genauen Form des Glieds, insbesondere dem Verlauf des Gliedumfangs entlang der Längsrichtung des Glieds, und zum anderen von der Strickbindung des Getricks abhängt. Insbesondere die stricktechnische Herstellung des distalen Endes des Stumpfstrumpfs, an dem bevorzugt ohne eine Naht ein kuppelförmig geschlossener Abschluss des Gestricks ausgebildet und gleichzeitig ein hoher Kompressionsdruck erzeugt werden soll, ist dabei schwierig.

Ausgehend von diesem Stand der Technik liegt der Erfindung die Aufgabe zu Grunde, ein Verfahren zur Herstellung eines Kompressionsgestricks zur Herstellung von gestrickten Stumpfstrümpfen mit kompressiver Wirkung und ein solches Kompressionsgestrick aufzuzeigen, mit denen sich in effizienter Weise komfortabel zu tragende Stumpfstrümpfe herstellen lassen, die beim Tragen keine Hautirritationen hervorrufen und einen gezielt einstellbaren Kompressionsdruck mit einem graduellen Druckverlauf erzeugen und individuell an die Form der amputierten Gliedmaßen, an denen der Stumpfstrumpf getragen werden soll, angepasst werden können.

Diese Aufgabe wird mit dem Verfahren mit den Merkmalen des Anspruchs 1 und dem Kompressionsgestrick des Anspruchs 9 gelöst. Bevorzugte Ausführungsformen des Verfahrens und des Kompressionsgestricks sind den abhängigen Ansprüchen zu entnehmen.

In dem Verfahren zur Herstellung eines zumindest im Wesentlichen schlauchförmigen Kompressionsgestricks, welches sich von einem offenen proximalen Ende zu einem geschlossenen distalen Ende erstreckt, erfolgt zunächst ein Stricken eines schlauchförmigen ersten Abschnitts eines Maschen bildenden Grundgestricks aus wenigstens einem Strickfaden entlang einer Strickrichtung, die von dem proximalen Ende zu dem distalen Ende des Kompressionsgestricks verläuft, mit einer Mehrzahl von in Strickrichtung aufeinanderfolgenden Maschenreihen, wobei in die Maschen des Grundgestricks in jeder Maschenreihe oder in jeder n-ten Maschenreihe mit $n \in \mathbb{N}$ und $n \geq 2$ ein elastischer Schussfaden über Fanghenkel eingebunden wird, und anschließend wird ein zweiter Abschnitt nahtloses an den ersten Abschnitt des Grundgestricks aus dem wenigstens einen Strickfaden entlang der Strickrichtung angestrickt, wobei in dem zweiten Abschnitt in Strickrichtung gesehen sukzessive die Anzahl der Maschen zumindest in einigen oder allen aufeinanderfolgenden Maschenreihen durch Umhängen von Maschen vermindert wird, wodurch in dem zweiten Abschnitt des Grundgestricks zum distalen Ende hin durch Maschenminderung ein kuppelförmig geschlossener Abschluss ausgebildet wird.

Durch die Maschenminderung in dem zweiten Abschnitt erhält dieser Abschnitt des Kompressionsgestricks eine zum distalen Ende hin spitz zulaufende Form. Dabei wird die Anzahl der Maschen in Umfangsrichtung des schlauchförmigen Gestricks in dem zweiten Ab schnitt durch Umhängen von Maschen in jeder Maschenreihe oder auch nur in ausgewählten Maschenreihen, bspw. in jeder zweiten, dritten oder vierten Maschenreihe, sukzessive vermindert, so dass der Umfang des schlauchförmigen Gestricks in Strickrichtung, d.h. vom offenen proximalen zum geschlossenen distalen Ende hin, immer kleiner wird, bis in der letzten gestrickten Maschenreihe am distalen Ende des Kompressionsgestricks nur noch eine einzige Masche verbleibt, welche zur Fixierung des Gestricks auf einer Nadel einer Flachstrickmaschine abgestrickt wird.

Durch die sukzessive Verringerung des Umfangs des schlauchförmigen Gestricks in dem zweiten Abschnitt bildet sich am distalen Ende des Kompressionsgestricks ein kuppelförmig geschlossener Abschluss aus. Dieser kuppelförmige Abschluss bildet bei einem aus dem Kompressionsgestricks hergestellten Stumpfstrumpf ein geschlossenes distales Ende aus, welches das Ende einer amputierten Körperextremität, bspw. eines Beinstumpfs, aufnehmen kann.

Durch die Maschenminderung in dem zweiten Abschnitt des Kompressionsgestricks kann die Form dieses Abschnitts passgenau an die Körperform der amputierten Körperextremität angepasst werden. Hierfür kann die amputierte Körperextremität bspw. mit einem Scanner erfasst und die Anzahl der Maschen in den einzelnen Maschenreihen des zweiten Abschnitts des Kompressionsgestricks an den erfassten Umfangsverlauf der Körperextremität in deren Längsrichtung angepasst werden. In entsprechender Weise kann auch die Anzahl der Maschen des Grundgestricks in dem ersten Abschnitt des Kompressionsgestricks an den Umfangsverlauf der Körperextremität angepasst werden. Auf diese Weise kann ein individuell auf die Form der Körperextremität eines Patienten angepasster Kompressionsartikel, bspw. ein individuell angepasster Stumpfstrumpf, hergestellt werden.

Da in dem Herstellungsverfahren ein elastischer Schussfaden in das Grundgestrick eingebunden wird, kann die vom Kompressionsgestrick beim Anlegen des Kompressionsartikels an eine (amputierte) Körperextremität erzeugte Kompressionsdruck genau eingestellt werden. Der Kompressionsdruck hängt dabei von der Spannung des elastischen Schussfadens ab, mit der dieser in das Grundgestrick eingebunden wird. Die Einbindung des elastischen Schussfadens erfolgt dabei durch Bildung von Fanghenkel. Durch die Spannung des Schussfadens und/oder über die Länge der Fanghenkel kann die Dehnbarkeit des Gestricks (und dadurch der auf eine Körperextremität erzeugbare Kompressionsdruck) eingestellt werden. Durch die Einbindung des elastischen Schussfadens in das Grundgestrick mit einer variablen Fadenspannung kann also insbesondere ein spezifisches Druckprofil des vom Kompressionsgestrick beim Anlegen an eine Körperextremität ausgeübten Kompressionsdrucks mit einem graduellen Druckverlauf erzeugt werden. Bevorzugt weist das Druckprofil dabei am distalen Ende des ersten Abschnitts des Kompressionsgestricks (bzw. an dem sich unmittelbar daran anschließenden proximalen Ende des zweiten Abschnitts) einen höheren Kompressionsdruck als am proximalen Ende des Kompressionsgestricks auf.

Bevorzugt wird der Schussfaden sowohl in dem ersten Abschnitt als auch in dem zweiten Abschnitt des Grundgestricks eingebunden, wobei der Schussfaden bspw. in jeder Maschenreihe oder in jeder n-ten Maschenreihe mit $n \in \mathbb{N}$ und $n \geq 1$ über Fanghenkel eingebunden werden kann. Bevorzugt ist eine Dichte des Schussfadens in den Maschenreihen des zweiten Abschnitts kleiner als eine Dichte des Schussfadens in den Maschenreihen des ersten Abschnitts. Die Dichte des Schussfadens in den Maschenreihen des Grundgestricks kann bspw. dadurch variiert werden, dass der Schussfaden zur Erzielung einer hohen Dichte in jede Maschenreihe und zur Erzielung einer niedrigeren Dichte nur in jeder zweiten, dritten oder vierten Maschenreihe, usw., eingebunden wird. Dabei ist es auch möglich, den Schussfaden unregelmäßig in die aufeinanderfolgenden Maschenreihen des Grundgestricks einzubinden bzw. unregelmäßig einzelne Maschenreihen ohne Einbindung des Schussfadens auszubilden. Auf diese Weise kann das Druckprofil des erzeugten Kompressionsdrucks entlang der Strickrichtung bzw. entlang der Längsrichtung einer Körperextremität, an die das Kompressionsgestrick angelegt wird, variiert werden, weil der erzeugte Kompressionsdruck von der Dichte des Schussfadens im Grundgestrick abhängt. Besonders Bevorzugt nimmt die Dichte des Schussfadens in dem kuppelförmigen Abschluss im Vergleich zur (durchschnittlichen) Dichte in den übrigen Zonen des zweiten Abschnitts ab, um am distalen Ende des Kompressionsgestricks, das bspw. bei der Verwendung als Stumpfstrumpf mit dem Ende einer amputierten Körperextremität in Berührung kommt, einen geringeren Kompressionsdruck zu erzeugen. Dadurch können Reizungen am sehr empfindlichen Stumpfende der amputierten Körperextremität vermieden werden.

Bevorzugt wird das Strickverfahren in einem unterbrechungsfreien Strickvorgang auf einer Flachstrickmaschine mit einem vorderen Nadelbett und einem hinteren Nadelbett durchgeführt, wobei das Kompressionsgestrick nahtlos gestrickt und eine vordere Lage des Kompressionsgestricks auf dem vorderen Nadelbett und eine der vorderen Lage gegenüberliegende hintere Lage auf dem hinteren Nadelbett gestrickt und die vordere Lage und die hintere Lage nahtlos durch Wechseln des mindestens einen Strickfadens vom vorderen Nadelbett auf das hintere Nadelbett, und umgekehrt, miteinander verstrickt werden, um das schlauchförmige Grundgestrick auszubilden, in welches der elastische Schussfaden mittels einer Schussfadeneinrichtung der Flachstrickmaschine durch Bildung von Fanghenkeln eingebunden wird. Durch das nahtlose Stricken des Kompressionsgestricks in einem unterbrechungsfreien Strickvorgang erhält das hergestellte Kompressionsgestrick einerseits einen guten Tragekomfort, der Hautreizungen an der Körperextremität des Trägers vermeidet und andererseits kann der Strickprozess sehr effizient und schnell durchgeführt werden.

Insbesondere die Maschenminderungen in dem zweiten Abschnitt des Kompressionsgestricks können dabei in Umhängevorgängen durch einen Versatz der Nadelbetten der Flachstrickmaschine auf sehr effiziente Weise erfolgen, wobei der Versatz der Nadelbetten um eine, zwei oder mehr Nadeln erfolgen kann. Entsprechend der Anzahl der Nadeln, um die die beiden Nadelbetten der Flachstrickmaschine beim jeweiligen Umhängevorgang zueinander versetzt werden, vermindert sich die Anzahl der Maschen in der betreffenden Maschenreihe des zweiten Abschnitts des Grundgestricks. Die Maschenminderung erfolgt dabei insbesondere in ausgewählten Maschenminderungszonen zwischen den Rändern der vorderen Lage und der hinteren Lage des Kompressionsgestricks, die jeweils mit dem Rand der jeweils gegenüberliegenden Lage durch Überführung des Strickfadens von dem vorderen Nadelbett auf das hintere Nadelbett, und umgekehrt, nahtlos verstrickt werden. In Maschenreihen mit einer sehr hohen Anzahl von Maschenminderungen sind bevorzugt mehrere solcher Maschenminderungszonen, an denen die Maschenminderungen in einer Maschenreihe erfolgen, im Abstand zueinander innerhalb einer Maschenreihe angeordnet. Dadurch kann die Gesamtzahl der Maschenminderung innerhalb einer Maschenreihe gleichmäßig über die Erstreckung der Maschenreihe in Maschenreihenrichtung bzw. in Umfangsrichtung des schlauchförmigen Gestricks, verteilt werden, wodurch ein einheitlicheres Strickbild entsteht und Aufwölbungen an den Rändern der vorderen und hinteren Lage des Kompressionsgestricks vermieden werden.

Zur Fixierung des Grundgestricks und um den kuppelförmigen Abschluss zu schließen wird der Strickfaden am distalen Ende des Kompressionsgestricks im Bereich der letzten belegten Nadel eines der beiden Nadelbetten der Flachstrickmaschine einmal oder mehrmals abgestrickt.

Zur Ausbildung des kuppelförmigen Abschlusses nimmt die Anzahl der Maschenminderungen in den Maschenreihen des zweiten Abschnitts des Grundgestricks bevorzugt in Strickrichtung gesehen zum distalen Ende des Kompressionsgestricks hin zu, bis lediglich eine letzte Nadel auf einem der Nadelbetten der Flachstrickmaschine als belegte Nadel verbleibt und der Strickfaden auf dieser letzten Nadel zur Fixierung des distalen Endes des Kompressionsgestricks ein- oder mehrfach abgestrickt wird. Insbesondere in den Maschenreihen des kuppelförmigen Abschlusses, in denen eine hohe Anzahl von Maschenminderungen erfolgt, werden bevorzugt mehrere Maschenminderungszonen vorgesehen, die jeweils im Abstand zueinander in einer Maschenreihe angeordnet sind.

Zur Fixierung des Schussfadens in dem Grundgestrick wird der von einer Schussfadenspule abgezogene Schussfaden zweckmäßig am distalen Ende des Kompressionsgestricks im Bereich der zuletzt gestrickten Maschen des Grundgestricks durch mehrere Fanghenkel im Grundgestrick verriegelt und danach von der Schussfadenspule abgeschnitten. Dies stellt sicher, dass sich der Schussfaden nicht aus dem Grundgestrick löst.

Mit dem erfindungsgemäßen Verfahren können Kompressionsgestricke, insbesondere zur Herstellung von gestrickten Stumpfstrümpfen mit kompressiver Wirkung, hergestellt werden, wobei das Kompressionsgestrick ein Maschen bildendes Grundgestrick mit einer Mehrzahl von aufeinanderfolgenden Maschenreihen umfasst und einen am proximalen Ende offenen schlauchförmigen ersten Abschnitt sowie einen sich in Strickrichtung daran anschließenden und nahtlos an den ersten Abschnitt angestrickten zweiten Abschnitt enthält, wobei der zweite Abschnitt an dem distalen Ende des Kompressionsgestricks einen kuppelförmig geschlossenen Abschluss ausbildet, der in dem zweiten Abschnitt durch sukzessive Verringerung der Anzahl der Maschen zumindest in einigen oder allen aufeinanderfolgend Maschenreihen des Grundgestricks zum distalen Ende hin ausgebildet ist, wobei in dem Grundgestrick, insbesondere im ersten und im zweiten Abschnitt, ein elastischer Schussfaden über Fanghenkel eingebunden ist.

Bevorzugt ist das Kompressionsgestrick insgesamt nahtlos ausgebildet. Dadurch können Hautreizungen und lokale Druckstellen, die bei Gestricken mit Nähten im Bereich der Nähte auftreten können, verhindert werden.

In einer bevorzugten Ausführungsform ist das Grundgestrick ein Rechts-/Links-Gestrick. Es können jedoch auch andere Strickbindungen zur Ausbildung des Grundgestricks verwendet werden, bspw. ein Rechts-/Rechts-Gestrick.

Der Schussfaden kann dabei in jeder Maschenreihe oder in jeder zweiten, dritten oder vierten Maschenreihe, u.s.w. in dem Grundgestrick eingebunden sein.

Der Strickfaden kann bspw. ein einfach umwundener Elasthanfaden oder ein elastisches Einfachgarn, ein gefachtes Garn oder ein Zwirn sein und der Schussfaden ist bevorzugt ein zweifach umwundener Elasthanfaden.

Das Kompressionsgestrick erzeugt bei Anlegen an eine amputierte Körperextremität eines Patienten einen Kompressionsruck auf die Körperextremität, bevorzugt mit einem graduellen Druckverlauf, wobei der Kompressionsdruck besonders bevorzugt in dem ersten Abschnitt vom proximalen Ende zum distalen Ende des Kompressionsgestricks hin graduell zunimmt. Zum geschlossenen Abschluss hin, also am distalen Ende des Kompressionsgestricks, nimmt der erzeugte Kompressionsdruck besonders bevorzugt wieder ab. Der erzeugte Kompressionsdruck weist dabei bspw. am Übergang des ersten Abschnitts zum zweiten Abschnitt ein Maximum auf und nimmt in dem zweiten Abschnitt zum distalen Ende des Kompressionsgestricks hin graduell ab. Dadurch können Reizungen des empfindlichen Endes des Stumpfs einer amputierten Körperextremität durch eine zu hohe Kompression im zweiten Abschnitt und insbesondere im Bereich des geschlossenen Abschlusses vermieden werden. Dies kann bspw. dadurch erzielt werden, dass die Dichte des Schussfadens in den letzten Maschenreihen des Grundgestricks zum distalen Ende hin immer weiter abnimmt. Am proximalen Ende des Kompressionsgestricks wird dabei zweckmäßig ein Kompressionsdruck von 10 mmHg bis 30 mmHg erzeugt und der maximale Kompressionsdruck liegt bevorzugt bei weniger als 35 mmHg.

Aus dem erfindungsgemäßen Kompressionsgestrick können nahtlose Kompressionsartikel für unterschiedliche medizinische Indikationen hergestellt werden, wie z.B. ein Stumpfstrumpf zum Anlegen an eine amputierte Körperextremität eines Patienten oder eine kompressive Kopfmaske zum Anlegen an den Kopf eines Patienten. Je nach Anwendungsfall wird dabei die Form des Gestricks dem Körperteil eines Patienten angepasst, insbesondere durch individuelle Maßanfertigung, wofür die Form des Körperteils bspw. mittels Scanner erfasst und bei der Herstellung des Kompressionsgestricks berücksichtigt wird, indem bspw. das Umfangsprofil des schlauchförmigen Grundgestricks und die Länge des Gestricks in Strickrichtung beim Strickvorgang eingestellt sowie etwaige Öffnungen (bspw. eine Gesichtsöffnung bei einer Gesichtsmaske) bereits beim Stricken ausgelassen werden.

Diese und weitere Vorteile sowie bevorzugte Merkmale der Erfindung ergeben sich aus den nachfolgend unter Bezugnahme auf die begleitenden Zeichnungen beschriebenen Ausführungsbeispielen, welche der Erläuterung der Erfindung dienen und den durch die nachfolgenden Ansprüche definierten Schutzbereich der Erfindung nicht beschränken. Die Zeichnungen zeigen:

**Fig. 1:** Darstellung von Strickbildern in Form einer technischen Patrone eines ersten Ausführungsbeispiels des erfindungsgemäßen Kompressionsgestricks, wobei in Figur 1A die ersten sieben Maschenreihen, in Figur 1B die sich daran anschließenden Maschenreihen 8 bis 16 und Figur 1C die das Gestrick in Strickrichtung abschließenden Maschenreihen 17 bis 27 zeigen;

**Fig. 2:** Darstellung eines Strickbilds in Form einer technischen Patrone eines zweiten Ausführungsbeispiels des erfindungsgemäßen Kompressionsgestricks, wobei in Figur 2 beispielhaft die ersten beiden Maschenreihen eines ersten Abschnitts des Gestricks dargestellt sind;

**Fig. 3:** Darstellung eines Strickbilds in Form einer technischen Patrone eines dritten Ausführungsbeispiels des erfindungsgemäßen Kompressionsgestricks, wobei in Figur 3 beispielhaft die ersten vier Maschenreihen eines ersten Abschnitts des Gestricks dargestellt sind;

**Fig. 4:** Photographische Darstellung eines Stumpfstrumpfs aus einem erfindungsgemäßen Kompressionsgestrick;

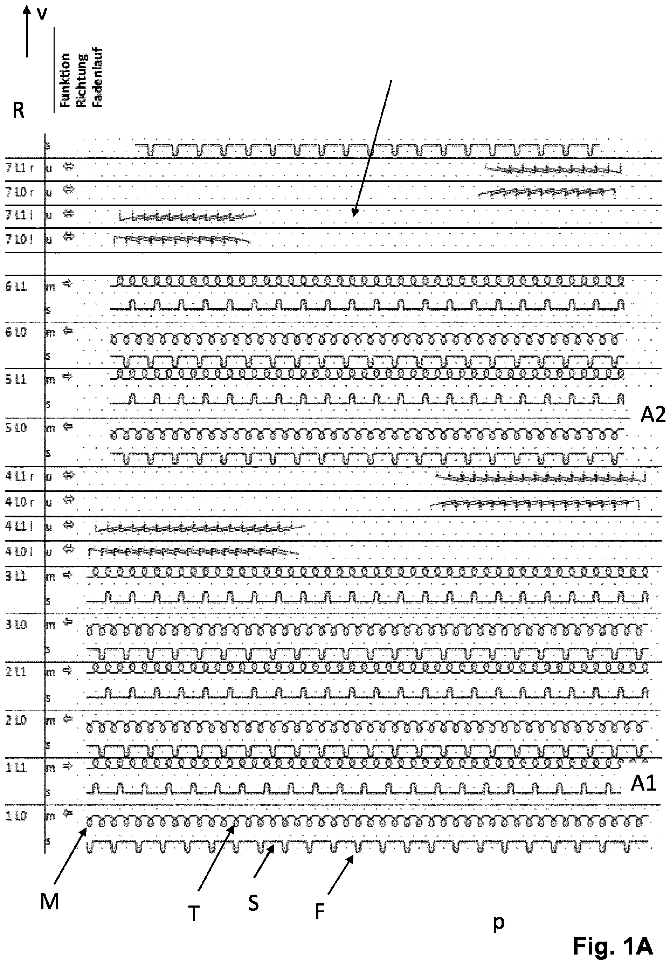

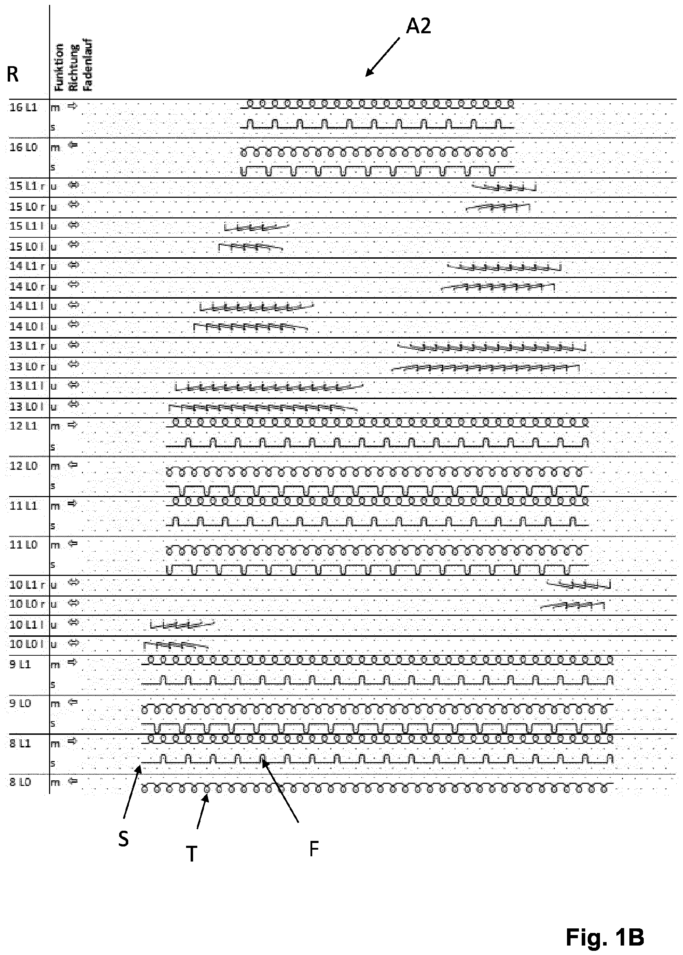

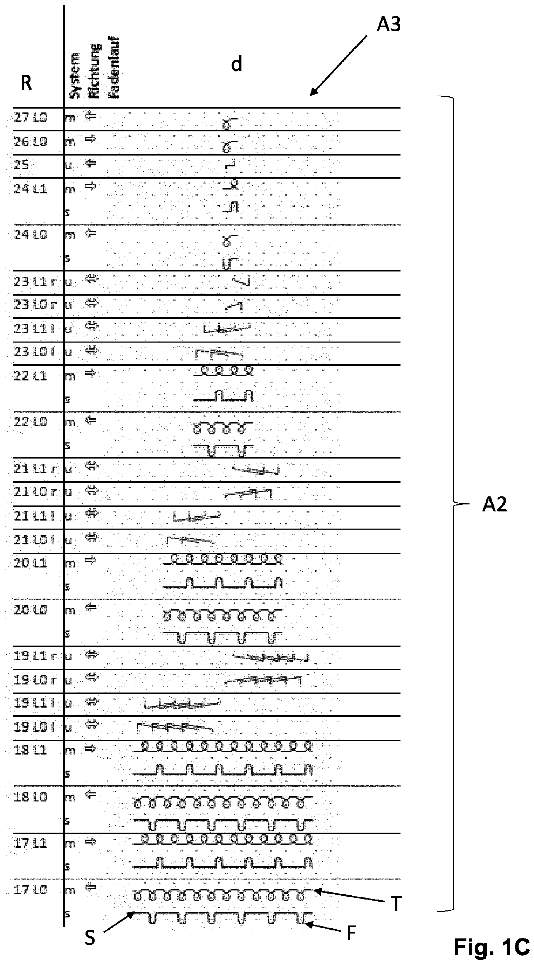

wobei in den Figuren 1 bis 3 folgende Symbole zu Darstellung der Strickbindungen eines Strickfadens T und eines elastischen Schussfadens S verwendet werden, wobei sich die Bezeichnungen "vorne" und "hinten" auf das vordere Nadelbett und das hintere Nadelbett einer Flachstrickmaschine beziehen, auf der das Kompressionsgestrick gestrickt wird:

| | |
|---|---|
| - - | Schussfaden: Flottung, vorne und hinten |
| ⌣ ⌢ | Schussfaden: Henkel/Fang, vorne und hinten |
| - - | Strickfaden (Flottung), vorne und hinten |
| ᶗ Ǭ | Strickfaden (Masche), vorne und hinten |
| ⌐ | Umhängen, von hinten nach vorne |
| ⌐ ⌐ | Umhängen, vorne 1 bzw. 2 Nadeln nach rechts hängen |
| ⌐ ⌐ | Umhängen, hinten 1 bzw. 2 Nadeln nach rechts hängen |

(fortgesetzt)

| | | |
|---|---|---|
| ⌐ | ⌐ | Umhängen, hinten 1 bzw. 2 Nadeln nach links hängen |
| ⌐ | ⌐ | Umhängen, vorne 1 bzw. 2 Nadeln nach links hängen |

In Figur 1 ist der Fadenverlauf eines ersten Ausführungsbeispiels für das erfindungsgemäße Kompressionsgestrick durch eine technische Patrone dargestellt, wobei das in Figur 1 gezeigte Kompressionsgestrick insgesamt 27 in einer Strickrichtung v aufeinander folgende Maschenreihen R umfasst, die in den Teilbildern der Figur 1 aufgeteilt sind, wobei Figur 1A die Maschenreihen 1 bis 7, Figur 1B die Maschenreihen 8 bis 16 und Figur 1C die Maschenreihen 17 bis 27 zeigen.

Das in Figur 1 gezeigte Kompressionsgestrick wird nahtlos und unterbrechungsfrei in einem durchgehenden Strickvorgang auf einer Flachstrickmaschine mit einem vorderen Nadelbett und einem hinteren Nadelbett gestrickt, wobei aus einem Strickfaden T eine vordere Lage L0 eines Grundgestricks M auf dem vorderen Nadelbett und eine der vorderen Lage gegenüberliegende hintere Lage L1 auf dem hinteren Nadelbett gestrickt und die vordere Lage mit der hinteren Lage nahtlos durch Wechseln des Strickfadens T an den Rändern der vorderen und der hinteren Lage vom vorderen auf das hintere Nadelbett, und umgekehrt, miteinander verstrickt werden, um ein schlauchförmiges Grundgestrick M auszubilden. Die vordere Lage ist in der technischen Patrone von Figur 1 in der jeweiligen Maschenreihe (in der linken Spalte neben der Maschenreihenzahl R) mit "L0" bezeichnet und die hintere Lage ist in jeder Maschenreihe mit "L1" bezeichnet. Die vorderen Lagen L0 und die hinteren Lagen L1 bilden durch das Verstricken der Randbereiche jeder vorderen Lage L0 mit dem Randbereich der hinteren Lage in derselben Maschenreihe und durch das Verbinden des Randbereichs einer hinteren Lage L1 mit dem Randbereich der vorderen Lage L0 der nachfolgenden Maschenreihe ein schlauchförmiges Grundgestrick M aus. In Figur 1 ist dabei die Richtung der Bewegung des Fadenführers entlang der Nadelbetten der Flachstrickmaschine in der Spalte "Richtung" mit einem Pfeil gekennzeichnet. Wie beispielsweise aus Figur 1A in Reihe 1 erkennbar, verläuft der Fadenführer beim Stricken der vorderen Lage L0 auf dem vorderen Nadelbett von rechts nach links und in der hinteren Lage L1 derselben Maschenreihe (Reihe 1) verläuft der Fadenführer auf dem hinteren Nadelbett von links nach rechts, u.s.w.. Dabei wird der Strickfaden T, aus dem das Grundgestrick M gestrickt wird, auf jeder Nadel der beiden Nadelbetten der Flachstrickmaschine zu einer Masche m verstrickt. Die Maschenbildungen des Strickfadens T sind dabei in Figur 1 in der Spalte "Funktion" jeweils mit dem Symbol "m" bezeichnet. In alternativen Ausführungsformen kann der Strickfaden T auch bspw. auf jeder zweiten Nadel des vorderen und des hinteren Nadelbetts der Flachstrickmaschine zu einer Masche m verstrickt und über dazwischenliegende Nadeln flott gelegt werden. Auch andere Grundgestrickbindungen sind dabei möglich, wobei der Strickfaden T insbesondere durch eine Kombination von Masche, Flottung oder Henkel auf jeder Nadel des vorderen und des hinteren Nadelbetts der Flachmaschine das Grundgestrick bildet. Bei dem Strickfaden T kann es sich bspw. um einen einfach umwundenen Elasthanfaden oder um ein Einfachgarn handeln. Der Strickfaden T kann auch als Doppelfaden oder als Zwirn ausgebildet sein und es kann zusätzlich zum Strickfaden T auch ein zusätzlicher Faden durch Plattieren in das Grundgestrick M eingebunden sein, wobei der Strickfaden T auf der rechten Warenseite liegt, während der zusätzliche Faden (plattierter Faden) auf der linken Warenseite liegt.

In das aus dem Strickfaden T ausgebildete Grundgestrick M wird mittels einer Schussfadeneinrichtung der Flachstrickmaschine ein elastischer Schussfaden S in das Grundgestrick M eingebunden. In dem in Figur 1 gezeigten Ausführungsbeispiel ist in jeder Maschenreihe R ein Schussfaden S eingebunden. Alternativ hierzu kann der elastische Schussfaden S auch nur in jeder zweiten, dritten oder vierten Maschenreihe R in das Grundgestrick M eingebunden werden. Dabei sind auch unperiodische Abläufe möglich, indem der elastische Schussfaden S beispielsweise zunächst in drei aufeinander folgenden Maschenreihen eingelegt, in einer vierten Maschenreihe ausgelassen, in der danach folgenden fünften Maschenreihe eingelegt und in der danach folgenden sechsten Maschenreihe wieder ausgelassen wird. Bei dem Schussfaden S kann es sich bspw. um einen doppelt umwundenen Elasthanfaden handeln.

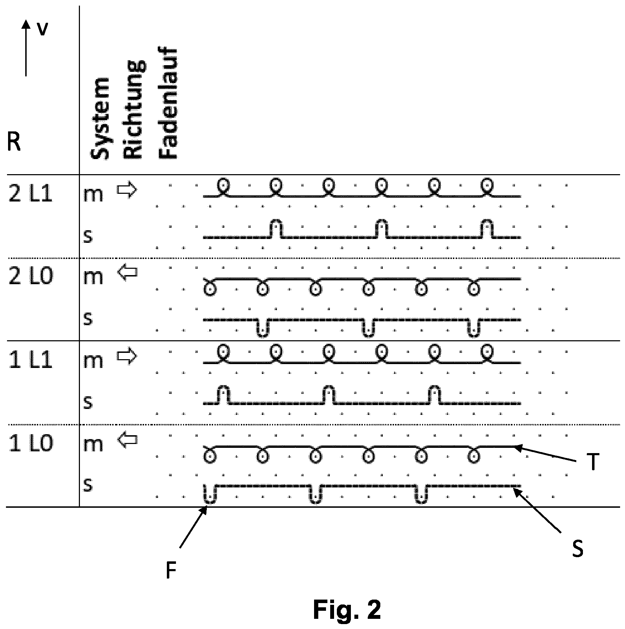

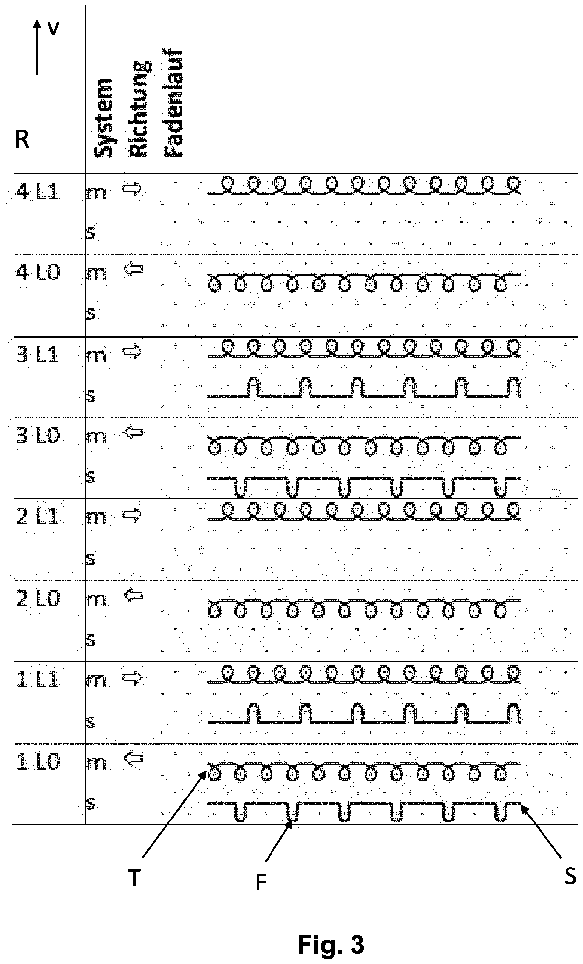

Die Einbindung des elastischen Schussfadens S in das Grundgestrick M, welche in den Zeichnungen der Figuren 1 bis 3 in der Spalte "Funktion" jeweils mit dem Symbol "s" bezeichnet ist, erfolgt durch Fanghenkel F, die auf bestimmten Nadeln des vorderen und des hinteren Nadelbetts der Flachstrickmaschine im Schussfadensystem S erzeugt werden. Zwischen zwei in Maschenreihenrichtung aufeinanderfolgende Fanghenkel F des Schussfadens S liegt der Schussfaden S flott. In dem in Figur 1 gezeigten Strickbild bildet der Schussfaden S auf jeder zweiten Nadel des vorderen und des hinteren Nadelbetts der Flachstrickmaschine einen Fanghenkel F und liegt entsprechend auf jeder zweiten, dazwischenliegenden Nadel flott. Andere Einbindungen des elastischen Schussfadens S in das Grundgestrick M sind möglich, wie in den Figuren 2 und 3 gezeigt. Bei dem in Figur 1 gezeigten Ausführungsbeispiel des Kompressionsgestricks handelt es sich um ein Rechts-/Links-Grundgestrick M mit 1:1 über Fanghenkel F hinterlegtem Schussfaden S.

Die in Figur 1 gezeigte Strickrichtung v verläuft von einem proximalen Ende p zu einem distalen Ende d des Kompressionsgestricks, wobei das proximale Ende p in Figur 1 in der ersten Maschenreihe 1 und das distale Ende d in Figur 1C in der letzten Maschenreihe (Reihe 27) dargestellt ist. Das proximale Ende p des Kompressionsgestricks von Figur 1 bildet dabei ein offenes Ende eines schlauchförmigen ersten Abschnitts A1 aus und das distale Ende d bildet einen kuppelförmig geschlossenen Abschluss A3 des Gestricks aus.

Der erste Abschnitt A1, der in Figur 1A gezeigt ist und hier die ersten drei Reihen 1 bis 3 des Gestricks umfasst, bildet ein schlauchförmiges Gestrick aus, welches auf dem vorderen Nadelbett und dem hinteren Nadelbett der Flachstrickmaschine eine bestimmte und in den aufeinanderfolgenden Maschenreihen des ersten Abschnitts A1 eine gleichbleibende (oder sich entlang der Strickrichtung v ändernde) Anzahl von Maschen m des Strickfadens T umfasst. Der erste Abschnitt A1 des Kompressionsgestricks kann jedoch noch weitere Maschenreihen mit jeweils einer gleichbleibenden oder sich entlang der Strickrichtung v ändernden Anzahl von Maschen m in jeder Maschenreihe R umfassen, welche hier nicht zeichnerisch dargestellt sind. Die Anzahl der Maschen m in den einzelnen Maschenreihen R des ersten Abschnitts A1 kann dabei unterschiedlich sein, um die Kontur des schlauchförmigen ersten Abschnitts A1 an die Körperform einer Körperextremität, an die das Kompressionsgestrick (bspw. in Form eines Stumpfstrumpfs) angelegt werden soll, anzupassen. Die Änderung der Maschenzahl in den einzelnen Maschenreihen R des ersten Abschnitts A1 kann hierfür insbesondere durch Maschenzunahmen oder Maschenminderungen erfolgen, welche zweckmäßig an den Rändern der vorderen Lage L0 und der hinteren Lage L1 des Grundgestricks M erfolgen.

An den ersten, schlauchförmigen Abschnitt A1 des Grundgestricks M schließt sich in Strickrichtung v, also zum distalen Ende d des Kompressionsgestricks hin, ein zweiter Abschnitt A2 mit einer sich zum distalen Ende d hin verjüngenden Kontur an. In dem in Figur 1 gezeigten Ausführungsbeispiel umfasst der zweite Abschnitt A2 die Maschenreihen 4 bis 27. Der elastische Schussfaden S ist dabei sowohl im ersten Abschnitt A1 als auch im zweiten Abschnitt A2 des Grundgestricks M eingebunden, wobei in dem gezeigten Ausführungsbeispiel die Einbindung des Schussfadens S im ersten Abschnitt A1 und im zweiten Abschnitt A2 gleichförmig ist, d.h. in beiden Abschnitten A1 und A2 ist der elastische Schussfaden S in jeder Maschenreihe über Fanghenkel F auf jeder zweiten Nadel der Nadelbetten der Flachstrickmaschine in das Grundgestrick M eingebunden.

Die Ausdehnung des Kompressionsgestricks in Umfangsrichtung, d.h. in Maschenreihenrichtung des Gestricks, wird in dem zweiten Abschnitt A2 des Kompressionsgestricks durch eine Maschenminderung mittels Umhängen des Strickfadens T unter Verwendung einer Umhängeeinrichtung sukzessive vermindert. Das Umhängen des Strickfadens T ist dabei in Figur 1 in der Spalte "Funktion" jeweils mit dem Symbol "u" bezeichnet. Durch das Umhängen u des Strickfadens T in ausgewählten Maschenreihen R des Grundgestricks M erfolgt eine Maschenminderung MM, d.h. eine Reduzierung der Anzahl der Maschen des Strickfadens T in der betreffenden Maschenreihe um eine vorgegebene Anzahl von Maschen. In dem in Figur 1 gezeigten Ausführungsbeispiel erfolgt die Maschenminderung bspw. in Reihe 4 des Gestricks sowohl auf dem vorderen Nadelbett als auch auf dem hinteren Nadelbett der Flachstrickmaschine und somit sowohl in der vorderen Lage L0 als auch in der hinteren Lage L1 des Gestricks. Die Nadeln des vorderen und des hinteren Nadelbetts der Flachstrickmaschine, auf denen der Strickfaden T zur Maschenminderung mit der Umhängeeinrichtung umgehängt wird, ist in Reihe 4 des Gestricks durch die dort gezeigten Klammersymbole gekennzeichnet. Jedes Klammersymbol symbolisiert dabei ein Umhängen u des Strickfadens T um eine vorgegebene Anzahl von Nadeln nach innen, also entgegen der ursprünglichen Bewegungsrichtung des Fadenführers, weshalb in der Spalte "Richtung" der Figur 1 in den Reihen des Gestricks, in denen ein Umhängen u des Strickfadens T erfolgt, jeweils ein Doppelpfeil eingezeichnet ist. Ein solches Umhängen u des Strickfadens T erfolgt in dem in Figur 1 gezeigten Ausführungsbeispiel in den Reihen 4, 7, 13 bis 15, 19, 21, 23 und 25. Durch die Maschenminderung kann die Kontur des Gestricks entlang der Strickrichtung v, also vom proximalen Ende p zum distalen Ende d hin, angepasst werden.

Die Maschenminderungen in den ausgewählten Maschenreihen des Gestricks auf weiter innen liegende Maschen des Grundgestricks erfolgt durch Umhängen u des Strickfadens T von dem aktiven Nadelbett der Flachstrickmaschine auf das gegenüberliegende Nadelbett, welches gerade inaktiv ist, und ein anschließendes Verschieben des inaktiven Nadelbetts gegenüber dem aktiven Nadelbett um eine vorgegebene Anzahl von Nadeln. Nach dem gegenseitigen Verschieben der beiden einander gegenüberliegenden Nadelbetten werden die Maschen des Strickfadens T von dem inaktiven Nagelbett zurück auf das aktive Nadelbett gehängt, wodurch aufgrund des Verschiebens der gegenüberliegenden Nadelbetten eine Maschenminderung, also eine Reduzierung der Anzahl der Maschen in der betreffenden Maschenreihe, erfolgt, in Abhängigkeit der Anzahl der Nadeln, um die die beiden Nadelbetten gegeneinander verschoben worden sind. Die Maschenminderungen erfolgen dabei in ausgewählten Maschenminderungszonen MM innerhalb ausgewählter Maschenreihen, wobei die Maschenminderungszonen MM zwischen den Rändern der vorderen Lage L0 bzw. der hinteren Lage L1 angeordnet sind und bevorzugt einen Abstand zu den Rändern der vorderen Lage bzw. der hinteren Lage aufweisen. Dabei können innerhalb einer Maschenreihe auch mehrere solcher Maschenminderungszonen MM im Abstand zueinander angeordnet werden.

Die Anzahl der auf diese Weise in den Maschenminderungszonen MM geminderten Maschenzahl kann dabei durch den Versatz der beiden gegenüberliegenden Nadelbetten zueinander gesteuert werden. In dem in Figur 1 gezeigten Ausführungsbeispiel erfolgt bspw. ein Versatz um zwei Nadeln. Es kann jedoch auch ein Versatz um nur eine Nadel oder auch um drei, vier oder mehr Nadeln erfolgen, mit einer entsprechenden Anzahl der Maschenminderungen um eine Masche oder um drei, vier oder mehr Maschen in der betreffenden Maschenreihe, in der eine Maschenminderung durch Umhängen u des Strickfadens erfolgt.

Die Maschenminderungen in den ausgewählten Maschenreihen des Gestricks auf weiter innen liegende Maschen des Grundgestricks kann auch durch Umhängen u des Strickfadens T von dem aktiven Nadelbett der Flachstrickmaschine auf ein Hilfsnadelbett erfolgen.

Das Umhängen u des Strickfadens T erfolgt dabei jeweils am Rand der betreffenden Maschenreihe R, also entweder am linken Rand l oder am rechten Rand r, wie aus den Reihen 4, 7, 13 bis 15, 19, 21 sowie 23 des Strickbilds von Figur 1 ersichtlich. Das Umhängen u des Strickfadens T erfolgt also entweder am linken Rand l der vorderen Lage L0 und am linken Rand der mit der vorderen Lage L0 verbundenen hinteren Lage L1, oder am rechten Rand r der vorderen Lage L0 und am rechten Rand r der damit verbundenen hinteren Lage L1. Die jeweiligen Außenkanten der vorderen Lage L0 und der hinteren Lage L1 werden dabei miteinander verbunden, indem der Strickfaden T vom vorderen Nadelbett auf das hintere Nadelbett der Flachstrickmaschine geführt wird, und umgekehrt, um die vordere Lage L0 mit der hinteren Lage L1 zu einem geschlossenen Schlauch miteinander zu verbinden.

Durch die Maschenminderung in ausgewählten Maschenreihen R des Gestricks kann die Ausdehnung des Gestricks in Maschenreihenrichtung entlang der Strickrichtung v variiert und dadurch die Kontur des Gestricks optimal der Körperform einer Körperextremität, an welche das Kompressionsgestrick zur Ausübung einer kompressiven Wirkung angelegt werden soll, angepasst werden. Die zur optimalen Anpassung der Form des Gestricks an die Körperform der Körperextremität erforderliche Maschenzahl in den einzelnen Maschenreihen R des Gestricks kann dabei zweckmäßig durch Einscannen der Körperform der Körperextremität und eine anschließende Berechnung der notwendigen Maschenzahl in den einzelnen Maschenreihen R erfolgen.

In dem zweiten Abschnitt A2 des Kompressionsgestricks, der sich in dem Ausführungsbeispiel von Figur 1 über die Maschenreihen 4 bis 27 erstreckt, ist durch die oben beschriebene Verfahrensführung beim Stricken auf der Flachstrickmaschine in Strickrichtung v gesehen sukzessive die Anzahl der Maschen m zumindest in einigen ausgewählten Maschenreihen R (in dem Ausführungsbeispiel von Figur 1 sind dies die Maschenreihen 4, 7, 10, 13 bis 15, 19, 21, 23 und 25) durch Umhängen u von Maschen m des Grundgestricks M vermindert, wodurch sich die Ausdehnung des Grundgestricks M in Maschenreihenrichtung entlang der Strickrichtung v, also zum distalen Ende d des Gestricks hin, auf Grund der Maschenminderung sukzessive vermindert. Dadurch entsteht zum distalen Ende d des Gestricks hin ein kuppelförmig geschlossener Abschluss A3. Der kuppelförmig geschlossene Abschluss A3 erstreckt sich in dem in Figur 1 und insbesondere in Figur 1C gezeigten Gestrick von Maschenreihe 23 bis zur letzten Maschenreihe 27. In Reihe 23 erfolgt dabei ein Umhängen u des Strickfadens T in der vorderen Lage L0 und der hinteren Lage L1 zunächst um jeweils zwei Maschen und anschließend erfolgt ein Umhängen u des Strickfadens T in der Maschenreihe 23 am rechten Rand r der vorderen Lage L0 und der hinteren Lage L1 um jeweils eine Masche. In der darauffolgenden Maschenreihe 24 wird der Strickfaden T sowohl auf der vorderen Lage L0 als auch auf der hinteren Lage L1 (also auf dem vorderen und dem hinteren Nadelbett der Flachstrickmaschine) zu einer einzigen verbliebenen Masche m verstrickt und der elastische Schussfaden S wird durch einen Fanghenkel F in diese Masche m eingebunden. In der darauffolgenden Maschenreihe 25 erfolgt ein letztes Umhängen u des Strickfadens T mittels der Umhängeeinrichtung um eine Masche m von hinten (also vom hinteren Nadelbett) nach vorne (also auf das vordere Nadelbett). In den beiden letzten Maschenreihen 26 und 27 wird der Strickfaden T jeweils auf dem vorderen Nadelbett (also in der vorderen Lage L0 des Gestricks) zu einer Masche m abgestrickt. Das Abstricken des Strickfadens T in einer letzten, verbliebenen Masche m auf dem vorderen Nadelbett der Flachstrickmaschine kann dabei beliebig oft erfolgen. Ein mehrfaches Abstricken stellt sicher, dass sich das Gestrick am geschlossenen, distalen Ende d nicht löst. Der elastische Schussfaden S wird an dieser Stelle durch eine oder mehrere Gestrickbindungen im Grundgestrick M verriegelt, insbesondere durch Fanghenkel F oder durch Maschenbildung, und danach von einer in der Schussfadeneinrichtung integrierten Schussfadenrolle, von der der Schussfaden S abgezogen wird, abgeschnitten.

Auf diese Weise kann ein schlauchförmiges Kompressionsgestrick mit einem elastischen Schussfaden S, der in das Grundgestrick M eingebunden ist, mit einem genau definierten Druckverlauf des vom Kompressionsgestrick auf eine Körperextremität ausgeübten Kompressionsdrucks hergestellt werden, wobei das schlauchförmige Gestrick ein offenes proximales Ende p und ein geschlossenes distales Ende d aufweist. Der vom Gestrick beim Anlegen an eine Körperextremität ausgeübte Kompressionsdruck kann dabei durch die Fadenspannung des Schussfadens S, mit der dieser in das Grundgestrick eingebunden wird, sowie die Länge der Fanghenkel F, durch die der Schussfaden S in das Grundgestrick eingebunden wird, gesteuert werden. Bevorzugt erzeugt das Kompressionsgestrick zumindest in dem ersten Abschnitt A1 einen graduell verlaufenden Kompressionsdruck mit zum proximalen Ende p hin abnehmendem Druck. Ein solches Kompressionsgestrick eignet sich insbesondere zur Verwendung als Stumpfstrumpf oder als Kopfmaske. Bei der Verwendung als Kopfmaske wird in das schlauchförmige Gestrick des ersten Abschnitts A1 eine Gesichtsöffnung eingestrickt. Dies kann durch Maschenminderung in ausgewählten Maschenreihen des ersten Abschnitts A1 erfolgen. Bei der Verwendung als Stumpfstrumpf kann in dem zweiten Abschnitt A2 der Druckverlauf zum distalen Ende d hin abnehmend ausgebildet werden, um eine zu hohe Druckbeanspruchung am distalen Ende einer amputierten Körperextremität zu verhindern. In dieser Ausführungsform weist das Gestrick am Übergang vom ersten Abschnitt A1 zum zweiten Abschnitt A2 ein Maximum des Kompressionsdrucks auf.

In Figur 2 ist der Fadenverlauf eines zweiten Ausführungsbeispiels für das erfindungsgemäße Kompressionsgestrick durch eine technische Patrone dargestellt, wobei das in Figur 2 gezeigte Kompressionsgestrick beispielhaft zwei Maschenreihen R (Reihe 1 und Reihe 2) des ersten Abschnitts A1 des erfindungsgemäßen Kompressionsgestricks zeigt, in denen das Grundgestrick M als Rechts-/Links-Multigauge-Gestrick mit einem darin in jeder Reihe R eingebunden elastischen Schussfaden S ausgebildet ist, wobei der Schussfaden S im Gegensatz zur Ausführungsform der Figur 1 auf jeder vierten Nadel der Nadelbetten der Flachstrickmaschine über einen Fanghenkel F in das Grundgestrick M eingebunden ist und zwischen zwei in einer Maschenreihe R aufeinanderfolgende Fanghenkel F über drei Nadeln flott liegt. An die beiden in Figur 2 gezeigten Maschenreihen schließen sich entsprechend ausgebildete Maschenreihen des zweiten Abschnitts A2 an, in denen über das oben beschriebene Umhängen u des Strickfadens T Maschenminderungen in ausgewählten Maschenminderungszonen MM entsprechend der Ausführungsform von Figur 1 erfolgen.

In Figur 3 ist der Fadenverlauf eines dritten Ausführungsbeispiels des erfindungsgemäßen Kompressionsgestricks durch eine technische Patrone dargestellt, wobei das in Figur 3 gezeigte Kompressionsgestrick beispielhaft vier Maschenreihen R (Reihen 1 bis 4) des ersten Abschnitts A1 des erfindungsgemäßen Kompressionsgestricks zeigt, in denen das Grundgestrick M als Rechts-/Links-Gestrick mit einem darin in jeder zweiten Reihe R eingebunden elastischen Schussfaden S ausgebildet ist, wobei der Schussfaden S, wie in der Ausführungsform der Figur 1, auf jeder zweiten Nadel der Nadelbetten der Flachstrickmaschine über einen Fanghenkel F in das Grundgestrick M eingebunden ist und zwischen zwei in einer Maschenreihe R aufeinanderfolgende Fanghenkel F über eine Nadeln flott liegt. In der Ausführungsform der Figur 3 ist der Schussfaden S in den ungeradzahligen Reihen R (Reihen 1, 3, etc.) des Grundgestricks M eingebunden und in den geradzahligen Reihen (Reihen 2, 4, etc.) befindet sich kein Schussfaden. An die vier in Figur 3 gezeigten Maschenreihen schließen sich entsprechend ausgebildete Maschenreihen des zweiten Abschnitts A2 an, in denen über das oben beschriebene Umhängen u des Strickfadens T Maschenminderungen in ausgewählten Maschenminderungszonen MM entsprechend der Ausführungsform von Figur 1 erfolgen.

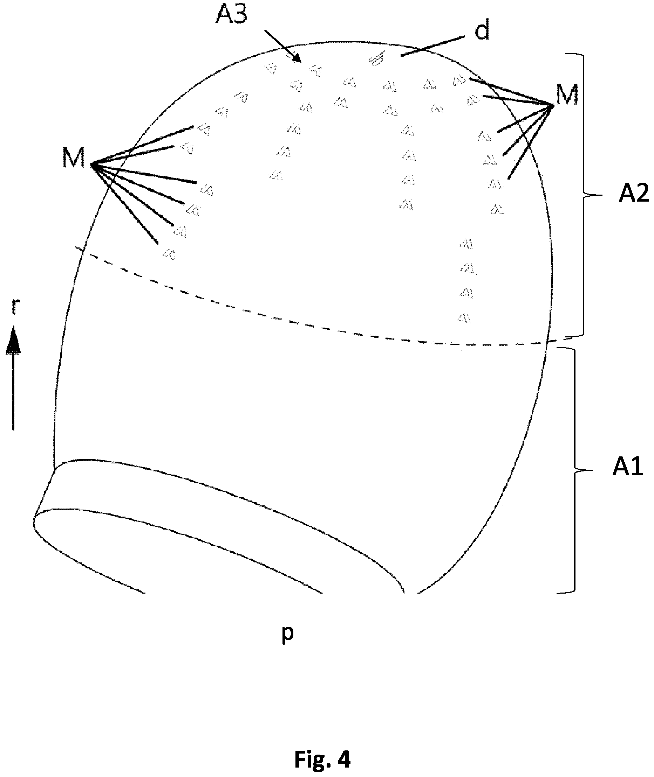

In Figur 4 ist ein Stumpfstrumpf zum Anlegen an eine amputierte Körperextremität, bspw. an ein amputiertes Bein, gezeigt, wobei der Stumpfstrumpf aus einem erfindungsgemäßen Kompressionsgestrick hergestellt ist, welches entlang der Strickrichtung v von einem proximalen Ende p zu einem distalen Ende d hin gestrickt ist und einen schlauchförmigen ersten Abschnitt A1 mit einer Öffnung am proximalen Ende p und einen sich daran anschließenden zweiten Abschnitt A2 mit einem kuppelförmig geschlossenen Abschluss A3 am distalen Ende d umfasst. In dem zweiten Abschnitt A3 sind durch die oben beschriebenen Umhängevorgänge beim Stricken ausgebildete Maschenminderungszonen enthalten, die im Gestrick auf der äußeren Warenseite erkennbar und in Figur 4 mit dem Zeichen "MM" bezeichnet sind. Zum distalen Ende d hin verjüngt sich der Umfang des zweiten Abschnitts A2 mit dem am distalen Ende d angeordneten geschlossenen Abschluss A3 aufgrund der Maschenminderungen in den Maschenminderungszonen MM immer weiter, bis zur letzten Masche, an der der Strickfaden und damit das aus dem Strickfaden gestrickte Grundgestrick durch ein mehrfaches Abstricken fixiert ist.

## Patentansprüche

1. Verfahren zur Herstellung eines zumindest im Wesentlichen schlauchförmigen Kompressionsgestricks, insbesondere zur Herstellung von gestrickten Stumpfstrümpfen mit kompressiver Wirkung, welches sich von einem offenen proximalen Ende (p) zu einem geschlossenen distalen Ende (d) des Kompressionsgestricks erstreckt, mit folgenden Schritten:

   • Stricken eines schlauchförmigen ersten Abschnitts (A1) eines Maschen (m) bildenden Grundgestricks (M) aus wenigstens einem Strickfaden (T) entlang einer Strickrichtung (v), die von dem proximalen Ende (p) zu dem distalen Ende (d) des Kompressionsgestricks verläuft, mit einer Mehrzahl von in Strickrichtung (v) aufeinanderfolgenden Maschenreihen (R), wobei in die Maschen (m) des Grundgestricks (M) in jeder Maschenreihe (R) oder in jeder n-ten Maschenreihe (M) mit n $\in \mathbb{N}$ und n $\geq$ 2 ein elastischer Schussfaden (S) über Fanghenkel (F) eingebunden wird,
   • Nahtloses Anstricken eines zweiten Abschnitts (A2) an den ersten Abschnitt (A1) des Grundgestricks (M) aus dem wenigstens einen Strickfaden (T) entlang der Strickrichtung (v), wobei in dem zweiten Abschnitt (A2) in Strickrichtung (v) gesehen sukzessive die Anzahl der Maschen (m) zumindest in einigen oder allen aufeinanderfolgenden Maschenreihen (R) durch Umhängen von Maschen (m) vermindert wird, wodurch in dem zweiten Abschnitt (A2) des Grundgestricks (M) zum distalen Ende (d) hin durch Maschenminderung ein kuppelförmig geschlossener Abschluss (A3) ausgebildet wird.

2. Verfahren nach Anspruch 1, **dadurch gekennzeichnet, dass** das Kompressionsgestrick nahtlos in einem unterbrechungsfreien Strickvorgang auf einer Flachstrickmaschine mit einem vorderen Nadelbett und einem hinteren Nadelbett gestrickt wird, wobei eine vordere Lage (L0) des Kompressionsgestricks auf dem vorderen Nadelbett und eine der vorderen Lage (L0) gegenüberliegende hintere Lage (L1) auf dem hinteren Nadelbett gestrickt wird und die vordere Lage und die hintere Lage an ihren jeweiligen Rändern nahtlos durch Wechsel des mindestens einen Strickfadens (T) vom vorderen Nadelbett auf das hintere Nadelbett und umgekehrt miteinander verstrickt werden, um ein schlauchförmiges Grundgestrick (M) auszubilden, in welches der elastische Schussfaden (S) mittels einer Schussfadeneinrichtung der Flachstrickmaschine mittels Fanghenkel (F) eingebunden wird.

3. Verfahren nach Anspruch 2, **dadurch gekennzeichnet, dass** die Maschenminderungen in dem zweiten Abschnitt (A2) des Kompressionsgestricks durch einen Versatz der Nadelbetten der Flachstrickmaschine erfolgt, wobei der Versatz der Nadelbetten um eine, zwei oder mehr Nadeln erfolgt.

4. Verfahren nach Anspruch 2 oder 3, **dadurch gekennzeichnet, dass** die Maschenminderung in ausgewählten Maschenminderungszonen (MM) in der vorderen Lage (L0) und/oder der hinteren Lage (L1) des Kompressionsgestricks zwischen den Rändern der vorderen Lage (L0) oder der hinteren Lage (L1) des Kompressionsgestricks erfolgt, wobei insbesondere in Maschenreihen (R) mit einer hohen Anzahl von Maschenminderungen bevorzugt mehrere Maschenminderungszonen (MM) in einem vorgegebenen Abstand zueinander innerhalb der Maschenreihe (R) angeordnet werden und jede Maschenminderungszone (MM) bevorzugt einen Abstand zu den Rändern der vorderen Lage (L0) oder der hinteren Lage (L1) aufweist.

5. Verfahren nach einem der voranstehenden Ansprüche, **dadurch gekennzeichnet, dass** der Strickfaden (T) am distalen Ende (d) des Kompressionsgestricks im Bereich der letzten belegten Nadel eines der beiden Nadelbetten der Flachstrickmaschine einmal oder mehrmals abgestrickt wird, um den kuppelförmigen Abschluss (A3) zu schließen.

6. Verfahren nach einem der voranstehenden Ansprüche, **dadurch gekennzeichnet, dass** die Anzahl der Maschenminderungen in den Maschenreihen (R) des zweiten Abschnitts (A2) des Grundgestricks (M) in Strickrichtung (v) gesehen zum distalen Ende (d) des Kompressionsgestricks hin zunimmt, bis lediglich eine letzte Nadel auf einem der Nadelbetten der Flachstrickmaschine als belegte Nadel verbleibt und der Strickfaden (T) auf dieser letzten Nadel zur Fixierung des distalen Ende (d) des Kompressionsgestricks ein- oder mehrfach abgestrickt wird.

7. Verfahren nach einem der voranstehenden Ansprüche, **dadurch gekennzeichnet, dass** der von einer Schussfadenspule abgezogene Schussfaden (S) am distalen Ende (d) des Kompressionsgestricks im Bereich der zuletzt gestrickten Maschen des Grundgestricks (M) durch ein oder mehrere Gestrickbindungselemente, insbesondere durch Fanghenkel, im Grundgestrick (M) verriegelt und danach von der Schussfadenspule abgeschnitten wird.

8. Verfahren nach einem der voranstehenden Ansprüche, **dadurch gekennzeichnet, dass** eine Dichte des Schussfadens in den Maschenreihen des zweiten Abschnitts bevorzugt kleiner ist als eine Dichte des Schussfadens in den Maschenreihen des ersten Abschnitts.

9. Kompressionsgestrick, insbesondere zur Herstellung von gestrickten Stumpfstrümpfen mit kompressiver Wirkung, wobei das Kompressionsgestrick ein Maschen (m) bildendes Grundgestrick (M) mit einer Mehrzahl von in einer Strickrichtung (v), die von einem proximalen Ende (p) zu einem distalen Ende (d) des Kompressionsgestrick verläuft, aufeinanderfolgende Maschenreihen (R) umfasst und einen am proximalen Ende (p) offenen schlauchförmigen ersten Abschnitt (A1) sowie einen sich in Strickrichtung (v) daran anschließenden und nahtlos an den ersten Abschnitt (A1) angestrickten zweiten Abschnitt (A2) enthält, wobei der zweite Abschnitt (A2) an dem distalen Ende (d) des Kompressionsgestricks einen kuppelförmig geschlossenen Abschluss (A3) ausbildet, der in dem zweiten Abschnitt (A2) durch in Strickrichtung (v) gesehen sukzessive Verringerung der Anzahl der Maschen zumindest in einigen oder allen aufeinanderfolgend Maschenreihen (R) des Grundgestricks (M) ausgebildet ist, **dadurch gekennzeichnet, dass** in das Grundgestrick (M) ein elastischer Schussfaden (S) über Fanghenkel (F) eingebunden ist.

10. Kompressionsgestrick nach einem der Ansprüche 9, **dadurch gekennzeichnet, dass** der Schussfaden in jeder Maschenreihe (R) oder in jeder zweiten Maschenreihe (R) oder in jeder dritten Maschenreihe (R) eingebunden ist.

11. Kompressionsgestrick nach einem der Ansprüche 9 oder 10, **dadurch gekennzeichnet, dass** die Dichte des Schussfadens (S) in den Maschenreihen (R) des Grundgestricks (M) im ersten Abschnitt (A1) höher als in dem zweiten Abschnitt (A2) ist.

12. Kompressionsgestrick nach einem der Ansprüche 9 bis 11, **dadurch gekennzeichnet, dass** der Strickfaden (T) ein einfach umwundener Elasthanfaden oder ein elastisches Einfachgarn, ein gefachtes Garn oder ein Zwirn ist und dass der Schussfaden (S) ein doppelt umwundener Elasthanfaden ist.

13. Kompressionsgestrick nach einem der Ansprüche 9 bis 12, **dadurch gekennzeichnet, dass** das Kompressionsgestrick bei Anlegen an eine amputierte Körperextremität eines Patienten einen Kompressionsruck mit einem graduellen Druckverlauf auf die Körperextremität ausübt, wobei der Kompressionsdruck zumindest in dem ersten Abschnitt (A1) vom offenen proximalen Ende (p) zum distalen Ende (d) des Kompressionsgestricks hin zunimmt.

14. Kompressionsgestrick nach Anspruch 13, **dadurch gekennzeichnet, dass** der Kompressionsdruck am Übergang des ersten Abschnitts (A1) zum zweiten Abschnitt (A2) ein Maximum aufweist und in dem zweiten Abschnitt (A2) zum distalen Ende (d) des Kompressionsgestricks hin abnimmt.

15. Kompressionsartikel, insbesondere Stumpfstrumpf zum Anlegen an eine amputierte Körperextremität eines Patienten oder kompressive Kopfmaske zum Anlegen an den Kopf eines Patienten, umfassend ein Kompressionsgestrick nach einem der Ansprüche 9 bis 14.

Fig. 1A

Fig. 1B

Fig. 1C

**Fig. 2**

**Fig. 3**

**Fig. 4**

Europäisches Patentamt
European Patent Office
Office européen des brevets

# EUROPÄISCHER RECHERCHENBERICHT

**Nummer der Anmeldung**

**EP 23 19 0726**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 4 840 635 A (SMITH WILLIAM B [US] ET AL) 20. Juni 1989 (1989-06-20) * Spalte 2, Zeilen 32-37; Abbildungen 2,6 * ----- | 9,15 | INV. D04B1/10 D04B1/18 D04B1/22 A61F2/78 |
| Y | DE 21 52 681 A1 (BENTLEY ENG CO LTD) 27. April 1972 (1972-04-27) * Seite 1, linke Spalte, Zeilen 46-51; Abbildungen 1-4 * * Seite 2, linke Spalte Zeile 39 – rechte Spalte, Zeile 9 * ----- | 1-15 | |
| Y | US 1 983 914 A (MCCANN JOHN A) 11. Dezember 1934 (1934-12-11) * Absätze [0009], [0012], [0013], [0015], [0016], [0026]; Abbildungen 1-3 * ----- | 1-15 | |
| A | DE 20 2017 103246 U1 (JULIUS ZORN GMBH [DE]) 3. September 2018 (2018-09-03) * Seite 11, Zeilen 2-20; Abbildungen 1-3 * ----- | 2-7 | |
| A | WO 2010/006051 A1 (KNIT RITE INC [US]; SMITH MARK W L [US]; DALBEY JEFFREY C [US]) 14. Januar 2010 (2010-01-14) * Absätze [0016], [0017], [0018], [0022] – [0026]; Abbildungen 1-3 * ----- | 1,10, 12-15 | RECHERCHIERTE SACHGEBIETE (IPC) D04B A61F |

Der vorliegende Recherchenbericht wurde für alle Patentansprüche erstellt

| Recherchenort | Abschlußdatum der Recherche | Prüfer |
|---|---|---|
| München | 9. Januar 2024 | Kirner, Katharina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 23 19 0726

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-01-2024

| Im Recherchenbericht angeführtes Patentdokument | Datum der Veröffentlichung | Mitglied(er) der Patentfamilie | Datum der Veröffentlichung |
|---|---|---|---|
| US 4840635 A | 20-06-1989 | KEINE | |
| DE 2152681 A1 | 27-04-1972 | DE 2152681 A1 | 27-04-1972 |
| | | ES 396303 A1 | 16-12-1974 |
| | | FR 2113133 A5 | 23-06-1972 |
| US 1983914 A | 11-12-1934 | KEINE | |
| DE 202017103246 U1 | 03-09-2018 | DE 202017103246 U1 | 03-09-2018 |
| | | EP 3412816 A1 | 12-12-2018 |
| | | US 2018347082 A1 | 06-12-2018 |
| WO 2010006051 A1 | 14-01-2010 | AU 2009268662 A1 | 14-01-2010 |
| | | BR PI0914117 A2 | 20-10-2015 |
| | | CA 2729315 A1 | 14-01-2010 |
| | | CN 102088938 A | 08-06-2011 |
| | | EP 2299955 A1 | 30-03-2011 |
| | | HK 1157621 A1 | 06-07-2012 |
| | | JP 5611200 B2 | 22-10-2014 |
| | | JP 2011527610 A | 04-11-2011 |
| | | KR 20110044975 A | 03-05-2011 |
| | | RU 2011104222 A | 20-08-2012 |
| | | US 2010005568 A1 | 14-01-2010 |
| | | US 2011146352 A1 | 23-06-2011 |
| | | WO 2010006051 A1 | 14-01-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7363778 B2